(19) Europäisches Patentamt
European Patent Office
Office européen des brevets

(11) **EP 0 548 233 B1**

(12) **EUROPEAN PATENT SPECIFICATION**

(45) Date of publication and mention
of the grant of the patent:
**22.10.1997 Bulletin 1997/43**

(21) Application number: **91917072.0**

(22) Date of filing: **07.08.1991**

(51) Int Cl.$^6$: **C07D 213/53**, C07D 213/48, C07D 213/38

(86) International application number:
**PCT/US91/05604**

(87) International publication number:
**WO 92/02507 (20.02.1992 Gazette 1992/05)**

(54) **PROCESSES FOR THE SYNTHESIS OF IMINES, ALDEHYDES, AND UNSYMMETRICAL SECONDARY AMINES**

VERFAHREN ZUR HERSTELLUNG VON IMINEN, ALDEHYDEN UND UNSYMMETRISCHEN SEKUNDÄREN AMINEN

PROCEDES DE SYNTHESE D'IMINES, D'ALDEHYDES ET D'AMINES SECONDAIRES NON SYMETRIQUES

(84) Designated Contracting States:
**BE CH DE FR GB IT LI NL SE**

(30) Priority: **10.08.1990 US 565956**

(43) Date of publication of application:
**30.06.1993 Bulletin 1993/26**

(73) Proprietor: **REILLY INDUSTRIES, INC.**
**Indianapolis Indiana 46204 (US)**

(72) Inventors:
• **GOE, Gerald**
**deceased (US)**
• **KEAY, James, G.**
**Indianapolis, IN 46256 (US)**
• **SCRIVEN, Eric, F., V.**
**Greenwood, IN 46142 (US)**
• **PRUNIER, Michael, L.**
**Vernon, IL 60061 (US)**
• **QUIMBY, Steven, J.**
**Carmel, IN 46032 (US)**

(74) Representative: **Bannerman, David Gardner et al**
**Withers & Rogers**
**4 Dyer's Buildings**
**Holborn**
**London, EC1N 2JT (GB)**

(56) References cited:
**US-A- 2 798 077** **US-A- 3 274 206**
**US-A- 4 358 446**

• **CHEMICAL ABSTRACTS, vol. 73, no. 15, 12 October 1970, Columbus, Ohio, US; abstract no. 76761B, P.N.RYLANDER ET AL.: 'HYDROGENATION OF NITRILES OVER PLATINUM METALS.' page 327 ; see abstract & ENGELHARD INDUSTRIES, TECHNICAL BULLETIN vol. 11, no. 1, 1970, NEWARK, N.J. SA 51605 030pages 19 - 24; cited in the application**
• **JOURNAL OF THE AMERICAN CHEMICAL SOCIETY. vol. 105, 1983, GASTON, PA US pages 5002 - 5011; S.-I. MURAHASHI ET AL.: 'CATALYTIC ALKYL GROUP EXCHANGE REACTION OF PRIMARY AND SECONDARY AMINES.' cited in the application see the whole document**
• **F. Zymalkowski, Katalytische Hydrierungen im Organisch-Chemischen Laboratorium, 256-259 (1965)**

**Description**

Background of the Invention

The present invention relates generally to the synthesis of imine intermediates which are useful in preparing aldehydes and unsymmetrical secondary amines, and also to the synthesis of unsymmetrical secondary amines. Specifically, the present invention relates to the preparation of stable imines by hydrogenating a mixture of a pyridinecarbonitrile and a primary amine in the presence of hydrogen and a rhodium-loaded hydrogenation catalyst, and the subsequent use of these imines as precursors of aldehydes and secondary amines.

Although the presence of imine intermediates in nitrile hydrogenations has been proposed and imines are suspected by-products from the hydrogenation of nitriles in the presence of primary amines, commercially useful quantities of selected imine products, as well as isolation or characterization of the same, from the hydrogenation of a pyridinecarbonitrile and a primary amine has been unknown. Rylander, P.N. and Hasbrouck, L., Eng. Tech. Bull., 11, 19 (1970), identified (by infrared) small quantities of uncharacterized imine as some unspecified portion of the miscellaneous compounds formed upon hydrogenation of benzonitrile and butylamine to produce high yields of various amines. No miscellaneous compounds were reported when a rhodium catalyst was used. Moreover, the Rylander and Hasbrouck work suggests that amine products are nearly quantitatively favored when nitrile hydrogenations take place in the presence of amines. In contrast, Applicants have discovered that pyridinecarbonitriles hydrogenated in the presence of rhodium and a primary amine unexpectedly form imines in good yield.

Imines have also been proposed as intermediates to explain the alkyl group exchange reactions of amines. Murahashi, S., et al., J. Am. Chem. Soc., 105, 5002 (1983).

Among other things, imines made according to Applicants' invention are beneficial intermediates and precursors. For instance, certain free aldehydes are known to be unstable and reactive. By way of illustration, 3-pyridinecarboxaldehyde oxidizes to give the solid carboxylic acid, i.e., niacin, upon exposure to ambient conditions. The solidification indicates a chemical change which makes the substance useless for its intended purpose. As a consequence, aldehydes of this sort are difficult to handle. Often referred to as masked aldehydes, imines can be hydrolyzed through nucleophilic attack to the corresponding aldehyde. This property of imines makes them ideal aldehyde precursors which may be stored until the aldehyde is needed. At that time, the imine is hydrolyzed to form the aldehyde. However, this same property has in the past caused some difficulties in imine synthesis. Under synthesis conditions, the imine may tend to hydrolyze to the aldehyde so that relatively little imine product is obtained.

Applicants have also discovered that yields of desired unsymmetrical secondary amines from nitrile hydrogenations are unexpectedly improved appreciably when stable imine intermediates are used according to the present invention. Nitrile hydrogenations are known to result in the production of symmetrical secondary amines. Schwoegler, E.J. and Adkins, H., J. Am. Chem. Soc., 61, 3499 (1939) taught that the ratio of primary to secondary amines in nitrile hydrogenations is seldom greater than 3 to 1. These researchers also taught that the yield of primary amine can be increased through addition of ammonia. When amines are added to the nitrile hydrogenation mixture, mixed secondary amines are produced in various amounts since the partially hydrogenated nitrile and the added amine compete to combine into secondary products. Accordingly, the mixed secondary amines which form detract from the yield of desired unsymmetrical product. The effect of this decrease in yield is especially great when the reactants are expensive.

Applicants' invention overcomes the problem of interfering mixed secondary amine by-products in the synthesis of unsymmetrical secondary amines. An intermediate imine made according to Applicants' process can be isolated and then manipulated using transimination techniques to displace one group of the imine with a more preferred group. The resulting imine may then be further hydrogenated to yield the selected unsymmetrical secondary amine in surprisingly good yield. Applicants are unaware of any reported reaction processes for the production of unsymmetrical secondary amines utilizing stable imine intermediates and subjecting such intermediates to transimination prior to final hydrogenation.

In addition to the selectivity with which unsymmetrical amine products can be made, enhanced efficiency and cost effectiveness are possible. In the typically less quantitative imine synthesis step of the reaction, a cheaper or more abundant or available primary amine reactant may be used to react with the nitrile. The amine used for the transimination step might then be more costly, less readily available, etc. valuable resources are consequently conserved.

The reductive intermolecular coupling of nitriles and primary aliphatic amines in molar ratios of about 1:1 is generally known to give unsymmetrical secondary amines, according to the following general reaction:

$$RCN + R'NH_2 + H_2 \rightarrow RCH_2NHR' + RCH_2NH_2.$$

These reductive couplings are known to take place between certain classes of nitriles and certain classes of amines. Applicants are unaware, however, of any instance where non-aliphatic nitriles have been reported to reductively couple with aromatic amines forming an unsymmetrical secondary amine product.

For example, in U.S. Patent No. 2,798,077, "Preparation of Methyl-(beta-picolyl)-Amine," issued to R. Schlapfer et al. in 1957, it is disclosed that 3-cyanopyridine can be hydrogenated in the presence of excess methylamine and a Raney nickel catalyst to yield N-methyl-3-picolylamine and beta-picolylamine. Separation of the beta-picolylamine is then required.

Aromatic amines that also contain nitrile groups have been reported to cyclize during intramolecular nitrile reduction to synthesize bicyclic systems, such as indoles. Rylander, P.N., Catalytic Hydrogenation Over Platinum Metals, p.215 (Academic Press, 1967).

Kindler, K. and Hesse, F., Arch. Pharm., 271, 439 (1933) disclosed the synthesis of secondary and tertiary amines by the hydrogenation of nitriles. Primary and secondary aliphatic amines were added to the reaction mixture to yield various amine products. An intermediate aldime was proposed to explain the variety of products obtained.

Aliphatic nitriles have been shown to reductively couple with aromatic and aliphatic amines. U.S. Patent No. 3,209,029 issued to Abramo et al. in 1965 discloses the synthesis of aminoalkyl-aromatic-ethylamines by the reduction of cyano-alkyl-aromatic-acetonitriles in the presence of a primary amine.

Further, certain catalysts are known to favor certain amine products in the hydrogenation of nitriles. In the hydrogenation of basic nitriles, including 3-cyanopyridine, to primary amines, formation of secondary symmetrical amine has been prevented by the use of a Raney nickel catalyst and the presence of ammonia. Various other catalysts including palladium and platinum catalysts, were reported to favor the formation of secondary amines. Huber, W., J. Am. Chem. Soc., 66, 876 (1944).

Rylander and Hasbrouck have discussed catalyst selectivity for certain amine products in the reductive coupling of benzonitrile and of aliphatic nitriles in the presence of aliphatic amines.. Rhodium on carbon, palladium on carbon and platinum on carbon were investigated for amine selectivity. Palladium was generally found to be a poor catalyst for reductive couplings to form amines, while rhodium and platinum were selective for certain secondary amines.

The reductive intermolecular coupling of aromatic or heteroaromatic nitriles and aromatic primary amines to yield unsymmetrical secondary amines has, however, remained unknown. In fact, Juday and Adkins reported that aniline did not couple with nitriles under conditions that were effective for coupling aliphatic amines with nitriles. Juday, R. E. and Adkins, H., J. Am. Chem. Soc., 77, 4559 (1955). In other work, Juday reported that no mixed secondary amine product formed from the addition of primary amines to the hydrogenation mixture of aromatic nitriles. The yield of tetrahydrohydrobenzamide did, however, increase. Juday, R. E., Proc. Montana Acad. Sci., 7-8, 84 (1947-48).

## Summary Of The Invention

Accordingly, Applicants have discovered a process for preparing a very useful class of imine compositions through the step of hydrogenating a mixture of a 3-cyanopyridine and a primary amine in the presence of a rhodium-loaded hydrogenation catalyst.

Applicant's have also discovered that yields of desired unsymmetrical secondary amines can be improved appreciably by first synthesizing an imine intermediate and then manipulating the imine intermediate to the desired amine. This preferred aspect of Applicants' discovery includes a process for preparing an unsymmetrical secondary amine composition, e.g., of the formula $RCH_2NHR^2$ where R is a 3-pyridyl group, by hydrogenating a mixture of 3-cyanopyridine and a first primary amine, e.g., of the formula R'NH2, in the presence of a rhodium-loaded hydrogenation catalyst to yield a first stable imine intermediate, e.g., having the formula $RCH=NR'$, transiminating the resulting stable imine intermediate with a second primary amine, e.g., having the formula $R^2NH_2$, to yield a second stable imine intermediate, e.g., having the formula $RCH=NR^2$ and then hydrogenating the second stable imine intermediate to thereby form the unsymmetrical secondary amine composition.

A still further embodiment of Applicants' discovery involves the preparation of an aldehyde from a stable imine precursor made according to the present process. The aldehydes are prepared by hydrogenating a mixture of a 3-cyanopyridine and a primary amine in the presence of a rhodium-loaded hydrogenation catalyst to form a stable imine intermediate, separating the hydrogenation catalyst from the imine intermediate and hydrolyzing the imine intermediate to the corresponding aldehyde.

Additional objects and advantages of the present invention will be apparent from the following description.

## Description of the Preferred Embodiments

A first aspect of the invention provides a process for preparing an imine composition having the formula $RCH=NR'$ wherein R is a 3-pyridyl group and R' is an aliphatic or aromatic group, comprising the step of:

forming a mixture of a 3-cyanopyridine and a primary amine of the formula $R'NH_2$;
causing the mixture to be at a temperature sufficiently high in the presence of a hydrogen source and a rhodium-loaded hydrogenation catalyst to form a stable imine composition of the formula $RCH=NR'$.

Preferably the rhodium-loaded catalyst is rhodium on carbon, most preferably about 5% rhodium on carbon. If so desired, the rhodium catalyst may be separated and recovered for later use using known recovery methods. In fact, rhodium on carbon has been recycled in the reductive coupling of 3-cyanopyridine with n-butylamine several times without a perceivable loss in catalytic activity.

The amine may be nearly any primary amine but preferably an aromatic or aliphatic amine. Examples of suitable amines are anilines and substituted anilines, n-butylamine and p-toluidine. The molar amount of primary amine present in the reaction mixture relative to the molar amount of nitrile is preferably at least about 1:1, although other molar ratios may prove advantageous. In the case where the imine is intended for use as a precursor or intermediate, the amine is preferably one which is relatively inexpensive, n-butylamine, for example. Further, if the amine used is a low-boiling one, then it may be boiled off to recover the imine product. Still further, the excess amine may be condensed and recovered for later use.

The pressures and temperatures used in the preferred process of this embodiment can be chosen for convenience and practicality, and can be thereby optimized in the usual fashion by those ordinarily skilled in this area. For example, hydrogen pressures ranging from about 50 to about 500 psig have been effectively used. The preferred pressure from testing thus far is about 50 psig. Similarly, temperatures ranging from about 20 to about 100°C have been used and proven suitable. The preferred temperature to date is one somewhat elevated above ambient, although this is not required.

If so desired, the reaction progress may be monitored by the pressure drop. After about one equivalent of hydrogen has been used, the reaction is preferably stopped by methods readily apparent to those skilled in the art. These methods include, but are not limited to, releasing the pressure and thus the hydrogen charge, lowering the temperature and/or removing the hydrogenation catalyst.

Isolation of the imine product may be by ordinary techniques, including recrystallization and distillation. The product might then be stored for later use as a reactant, end-product, precursor, etc. When intended as an aldehyde precursor, described below, it is often preferable to isolate the stable imine for storage until the aldehyde is needed. This is so since the imine (or masked aldehyde) is often appreciably more stable than the corresponding aldehyde and can be stored for appreciably longer times without significant degradation.

The above embodiment is useful for the synthesis of imines of general application as intermediates, precursors or end-products. As noted, applicants have found the imine intermediate to be superior for the production of aldehydes and unsymmetrical secondary amines. Thus, a second aspect of applicants' invention concerns the synthesis of unsymmetrical secondary amines by first synthesizing an imine precursor according to the first aspect of the invention. The imine precursor may then be manipulated to the desired amine. Transimination is included in the preferred manipulation technique. This reaction sequence results in unexpectedly improved yields of the desired amine, with corresponding decreases in undesired mixed side products.

The second aspect of the invention provides a process for preparing an unsymmetrical secondary amine composition of the formula $RCH_2NHR^1$ where R is 3-pyridyl group and $R^1$ is an aliphatic or aromatic group, comprising the steps of:

causing a mixture of 3-cyanopyridine and a first primary amine to react by the process according to the first aspect of the invention to yield a first stable imine intermediate;
contacting the stable imine intermediate with a second primary amine of the formula $R^2NH_2$, where $R^2$ is an aromatic group, and causing the mixture so formed to be at a temperature sufficiently high and/or sufficiently acidic to yield a second stable imine intermediate; and
contacting the second stable imine intermediate with a hydrogen source in the presence of a suitable hydrogenation catalyst at elevated pressure and at a temperature sufficiently high to thereby form the secondary amine composition of the formula $RCH_2NHR^2$.

The rhodium loaded hydrogenation catalyst may be rhodium on carbon, and may be recovered after the hydrogenating step, but before the transanimation step.

Preferably the second primary amine is a polyhaloaniline Such as a polyfluoroaniline, especially 2,4-difluoroaniline.

As discussed, by-products, such as symmetrical secondary amines, detract from the yield of the desired unsymmetrical secondary amine. The yield loss to such by-products might be serious, depending on the relative electronic effects of the R and R' groups. In the second aspect of the present invention, usable product can be recovered from what were previously undesirable symmetrical by-products by combining reductive intermolecular coupling of nitriles and amines with transimination (also known as metathesis) techniques to synthesize the desired unsymmetrical secondary amines. In so doing, it has also been discovered that N-(2,4-difluorophenyl)-3-picolylamine, and other N-substituted 3-picolylamines that were not heretofore as easily prepared in acceptable yields by the direct reductive intermolecular coupling of the appropriate nitriles and primary aromatic amines, can be prepared in greatly increased yields.

In general, the initial step of the reaction sequence forms an imine of the general formula RCH=NR'. Using the concept of transimination, the stable imine intermediary may, be manipulated with a second primary amine, $R^2NH_2$ to form a second stable intermediary $RCH=NR^2$ and $R'NH_2$. The $R'NH_2$ might then be removed by distillation or acidification and extraction, and recovered, thereby enriching the reaction mixture in the desired second imine. The reaction mixture, enriched in the exemplary stable intermediary precursor RCH=NR$^2$, can then be further hydrogenated to reduce the precursor to the unsymmetrical secondary amine RCH$_2$NHR$^2$. This entire exemplary reaction process may be represented in three substeps, as follows:

(a)      RCN + R'NH$_2$ + H$_2$ → RCH=NR'

(b)      RCH=NR' + R$^2$NH$_2$ → RCH=NR$^2$ + R'NH$_2$

(c)      RCH=NR$^2$ + H$_2$ → RCH$_2$NHR$^2$

wherein R is non-aliphatic, R' may be aliphatic or non-aliphatic, and R$^2$ may be aromatic but is not attached to R or R'.

Substep (a) proceeds according to the first embodiment of Applicants' invention. If desired or necessary, the intermediate RCH=NR' (or the intermediate RCH=NR$^2$) can be isolated by the usual techniques of distillation or recrystallation, but this is not required.

Substep (b), the transimination step, will proceed under the influence of electronic effects to completion. If the R'NH$_2$ amine has a suitably low boiling point (preferably lower than that of the solvent utilized, if any, but at least lower than that of the second amine R$^2$NH$_2$), it may be driven from the reaction solution by heating, leaving the preferred intermediate, RCH=NR$^2$, in the reaction mixture. Another method of driving the second substep to the desired degree of completion is to rely on the differences in basicity of aliphatic and aromatic amines. This method has the most application when the first primary amine is aliphatic and the second is aromatic. Adding acid to the mixture of the RCH=NR' intermediary, and the aromatic amine, ArNH$_2$, generates the anil, RCH=NAr, and the more basic R'NH$_2$ as an acid salt. This equilibrium has been demonstrated by Singh et al., J. Chem. Soc. Perkin I, 2091 (1986). Further, concentrated HCL or another acid may be added to promote the transimination.

The hydrogenating step may be in the presence of a solvent, such as the first primary amine. The solvent may be separated and recovered before transaminating.

An additional aspect of the second embodiment is Applicants' discovery that the initial hydrogenation reaction (a) can be enhanced appreciably if an aliphatic amine, R'NH$_2$, is utilized. Then the manipulation of the RCH=NR' intermediary with transimination techniques and a second primary amine R$^2$NH$_2$ results in greatly improved yields of the amine precursor RCH=NR$^2$, and thus the unsymmetrical secondary amine products. The advantages of utilizing an aliphatic amine in substep (a) include: (1) aliphatic amines are effectively coupled in a lower molar ratio to the nitrile (1:1) than, for example, aromatic amines such as 2,4-difluoroaniline or other amines with pKa's lower than R'NH$_2$; (2) aliphatic amines are generally cheaper starting materials; (3) overall yields are improved because of the greater yields achievable in the first hydrogenation, (4) the kinetics of the first hydrogenation are improved with aliphatic amines versus aromatic amines; (5) many different unsymmetrical secondary amines can be prepared from the same initial intermediate RCH=NR' by transimination with various second primary amines, while non-aliphatic groups might be harder to displace; (6) amines with functional groups that would not survive initial reduction, such as -CHO and -CN, can be substituted in the transimination step (if they would survive the conditions of that step); and (7) costly primary aromatic amines can be utilized more efficiently in the transimination step rather than the initial hydrogenation step.

In any event, practically any amine R'NH$_2$ can be used in substep (a). Preferred primary amines to date have been aliphatic amines such as n-butylamine, t-butylamine and cyclohexylamine, with n-butylamine being particularly preferred since it is cheap and low boiling. In the initial hydrogenation step, aliphatic amines are preferably present in about a molar ratio of 1:1 relative to the nitrile. On the other hand, aromatic amines are preferably present in excess of about 1:1. The transimination step (b) typically requires about a 1:1 molar ratio of imine to second primary amine almost regardless of the second amine's character.

The catalyst for the third substep, or final hydrogenation, may be any suitable hydrogenation catalyst. Such catalysts are commonly precious metal catalysts although others may be suitable. Preferred catalysts to date include palladium, platinum and rhodium containing catalysts, especially platinum on carbon, palladium on carbon or barium sulfate, rhodium on carbon, or rhodium on alumina.

Moreover, the reaction conditions may be those which are typically used in imine hydrogenations, including elevated pressure from the hydrogen charge and elevated temperature. Of course, the conditions may be optimized depending upon the desired reaction rate, etc.

A third aspect of the invention provides a process for preparing 3-pyridylaldehyde comprising the steps of:

forming a mixture of a 3-cyanopyridine and a primary amine and causing them to react by the process according to Claim 1, to form a stable imine intermediate of the formula RCH=NR' wherein R is a 3-pyridyl group;
separating the hydrogenation catalyst from the imine intermediate; and
hydrolysing the imine intermediate to a corresponding 3-pyridylaldehyde.

The rhodium-loaded catalyst may be rhodium on carbon, which may be about 5% on carbon.

Preferably, the primary amine is an aliphatic amine.

The hydrogentating step may be in the presence of a solvent which may be the primary amine.

Imines are known aldehyde precursors. Imines are however, more stable than aldehydes and, consequently, make a desirable storage medium. When it becomes time to use the aldehyde, the imine is simply hydrolyzed in the presence of a base. Particularly advantageous uses of the present invention are described in Examples 5 and 6. In these Examples, N-butyl-3-pyridylmethyleneimine is hydrolyzed to 3-pyridinecarboxaldehyde, a particularly noxious, although useful, aldehyde. Example 5 illustrates a base hydrolysis, while Example 6 is an acid hydrolysis.

For the purpose of promoting a better understanding of the Drocesses of the Applicants' invention, reference will be made in the Examples that follow to specific instances of their use. These Examples are for illustration only, and no limitation of the scope or breadth of the Applicants' invention is thereby intended.

## EXAMPLE 1

### Preparation of N-(2,4-difluorophenyl)-N-(3-pyridylmethylene)imine

A mixture of 3-cyanopyridine (5.2 g), 2,4-difluoroaniline (96.7 g) and 5% rhodium on carbon (0.5 g) was treated with hydrogen at 60 psig and 30 deg C in a rocking autoclave for thirteen hours. The catalyst was removed by filtration and the filtrate was analyzed by glc to show 3.6 g of N-(2,4-difluorophenyl)-N-(3-pyridylmethylene)imine.

## EXAMPLE 2

### Preparation of N-(4-methylphenol)-N-(3-pyridylmethylene)imine

A mixture of 3-cyanopyridine (20.8 g), 100 mL of methanol, p-toluidine (21.4 g), and 5% rhodium on carbon (1.0 g) was treated with hydrogen at 50 psig and 50 deg C for eight hours in a rocking autoclave. The catalyst was removed by filtration and the filtrate was analyzed by glc to show 17.7 g of N-(4-methylphenyl)-N-(3-pyridylmethylene)imine.

## EXAMPLE 3

### Preparation of N-(3-pyridylmethyl)-N-(3-pyridylmethylene)imine, transimination to N-(2,4-difluorophenyl)-N-(3-pyridylmethylene)imine, and reduction to N-(2,4-difluorophenyl)-N-(3-pyridylmethyl)amine

(a) A mixture of 3-cyanopyridine (72.8 g), 350 mL of methanol, 2,4-difluoroaniline (270.9 g), and 5% rhodium on carbon (3.5 g) was treated with hydrogen at 50 psig and 30 deg C for 30 hours. The catalyst was removed by filtration and the filtrate was analyzed by glc to show 30.4 g of N-(2,4-difluorophenyl)-N-(3-pyridylmethylidine)imine, 2.0 g of N-(2,4-difluorophenyl)-N-(3-pyridylmethyl)amine, and 50.2 g of N-(3-pyridylmethyl)-N-(3-pyridylmethylene)imine. The methanol was removed by distillation.

(b) Concentrated hydrochloric acid (60 mL) in distilled water (200 mL) was added to the residue to promote transimination. The organic layer was separated, washed with 50 mL of 10% aqueous sodium hydroxide, and dried over magnesium sulfate.

(c) The resulting material, 350 mL of methanol, and 5% palladium on carbon (5.0 g) were treated with hydrogen at 400 psig and 35 deg C for two hours in a rocking autoclave. The catalyst was removed by filtration and the solution was distilled to give 59.0 g of N-(2,4-difluorophenyl)-N-(3-pyridylmethyl) amine.

## EXAMPLE 4

### Preparation of N-(butyl)-N-(3-pyridylmethylene)imine, transimination to N-(2,4-difluorophenyl)-N-(3-pyridylmethylene)imine, and reduction to N-(2,4-difluorophenyl)-N-(3-pyridylmethyl)amine

(a) A mixture of 3-cyanopyridine (104.0 g), 500 mL of methanol, n-butylamine (73.0 g), and 5% rhodium on carbon catalyst (10.0 g) was treated with hydrogen at 50 psig and 35 deg C for seven hours in a rocking autoclave. The catalyst was removed by filtration and the filtrate analyzed by glc to show 99.7 g of N-(n-butyl)-N-(3-pyridylmethylene)imine and 18.2 g of N-(3-pyridylmethyl)-N-(3-pyridylmethylene)imine. Methanol and excess n-butylamine were removed by distillation.

(b) To the resulting concentrate were added methylene chloride (600 mL), water (300 mL), concentrated hydrochloric acid (75 mL), and 2,4-difluoroaniline (117 g). The mixture was stirred for three hours at room temperature. The methylene chloride was separated, washed with 10% aqueous sodium hydroxide, and dried over magnesium sulfate. The solvent was removed by evaporation and the resulting brown solid was dissolved in 450 mL of methanol.

(c) To this was added 5% palladium on carbon catalyst (6.0 g) and the mixture was treated with hydrogen at 400 psig and 32 deg C for two hours in a rocking autoclave. The catalyst was removed by filtration and the filtrate was distilled to give 21.7 g (30%) of N-(2,4-difluorophenyl)-N-(3-pyridylmethyl)amine.

## EXAMPLE 5

### Preparation of 3-Pyridinecarboxaldehyde by base hydrolysis

N-butyl-N-(3-pyridylmethylene)imine (18.6 g., 0.1 mol), made according to step (a) of Example 4 above, was heated at reflux in water (100 ml) in the presence of NaOH (0.2 mol). Butylamine was recovered by distillation. The solution was cooled and the product extracted into dichloromethane (3 x 50 mls). After drying over magnesium sulfate, the combined extracts were distilled and 3-pyridinecarboxaldehyde was obtained as a clear colorless liquid.

## EXAMPLE 6

### Preparation of 3-Pyridinecarboxaldehyde by acid hydrolysis

N-butyl-N-(3-pyridylmethylene)imine (18.6 g., 0.1 mol), made according to step (a) of Example 4 above, was heated at reflux in water (100 ml) in the presence of HCl (1.1 mol) until no further starting material was detected by thin layer chromatography. After cooling and pH adjustment, the solution was extracted with dichloromethane (3 x 50 mls). After combination, the organic extracts were distilled and dried. 3-pyridinecarboxaldehyde was distilled at 95-97 degrees C and 15 mm Hg as a clear colorless liquid.

## Claims

1. A process for preparing an imine composition having the formula RCH=NR' wherein R is a 3-pyridyl group and R' is an aliphatic or aromatic group, comprising the step of:

    forming a mixture of a 3-cyanopyridine and a primary amine of the formula R'NH$_2$;
    causing the mixture to be at a temperature sufficiently high in the presence of a hydrogen source and a rhodium-loaded hydrogenation catalyst to form a stable imine composition of the formula RCH=NR'.

2. The process of Claim 1 in which the rhodium-loaded catalyst is rhodium on carbon.

3. The process of Claim 2 in which the rhodium-loaded catalyst is about 5% rhodium on carbon.

4. The process of Claim 1 in which the primary amine is an aromatic amine.

5. The process of Claim 1 in which the primary amine is an aliphatic amine.

6. The process of Claim 1 and further comprising the steps of separating and recovering the hydrogenation catalyst.

7. A process for preparing an unsymmetrical secondary amine composition of the formula RCH$_2$NHR$^1$ where R is a 3-pyridyl group and R$^1$ is an aliphatic or aromatic group, comprising the steps of:

    causing a mixture of 3-cyanopyridine and a first primary amine to react by the process according to Claim 1 to yield a first stable imine intermediate;
    contacting the stable imine intermediate with a second primary amine of the formula R$^2$NH$_2$, where R$^2$ is an aromatic group, and causing the mixture so formed to be at a temperature sufficiently high and/or sufficiently acidic to yield a second stable imine intermediate; and
    contacting the second stable imine intermediate with a hydrogen source in the presence of a suitable hydrogenation catalyst at elevated pressure and at a temperature sufficiently high to thereby form the secondary amine composition of the formula RCH$_2$NHR$^2$.

8. The process of claim 7 in which the second primary amine is a polyhaloaniline.

9. The process of claim 8 in which the polyhaloaniline is a polyfluoroaniline.

10. The process of claim 9 in which the polyfluoroaniline is 2,4-difluoroaniline.

11. The process of claim 7 in which the rhodium-loaded hydrogenation catalyst is rhodium on carbon.

12. The process of claim 7 in which said hydrogenating the second imine intermediate is in the presence of a hydrogenation catalyst selected from the group consisting of platinum on carbon, palladium on carbon or barium sulfate, rhodium on carbon, and rhodium on alumina.

13. The process of claim 7 and further comprising the steps of separating and recovering the rhodium-loaded hydrogenation catalyst after said hydrogenating but before said transiminating.

14. The process of claim 13 in which said hydrogenating is in the presence of a solvent.

15. The process of claim 14 in which the solvent is the first primary amine.

16. The process of claim 14 and further comprising the step of removing the solvent after said separating and recovering and before said transiminating.

17. The process of claim 16 in which said transiminating is in the presence of hydrochloric acid.

18. The process of Claim 7 in which said first primary amine is a primary aliphatic amine.

19. A process for preparing 3-pyridylaldehyde comprising the steps of:

forming a mixture of a 3-cyanopyridine and a primary amine and causing them to react by the process according to Claim 1, to form a stable imine intermediate of the formula RCH=NR' wherein R is a 3-pyridyl group;

separating the hydrogenation catalyst from the imine intermediate; and

hydrolysing the imine intermediate to a corresponding 3-pyridylaldehyde.

20. The process of Claim 19 in which the rhodium-loaded catalyst is rhodium or carbon.

21. The process of Claim 20 in which the rhodium-loaded catalyst is about 5% rhodium or carbon.

22. The process of Claim 19 in which the primary amine is an aliphatic amine.

23. The process of Claim 22 in which said hydrogenating is in the presence of a solvent.

24. The process of Claim 23 in which the solvent is the primary amine.

**Patentansprüche**

1. Verfahren zur Herstellung einer Iminverbindung mit der Formel RCH=NR', wobei R eine 3-Pyridylgruppe und R' eine aliphatische oder aromatische Gruppe ist, mit den Schritten:

Bildung eines Gemischs aus einem 3-Cyanopyridin und einem primären Amin der Formel R'NH$_2$;

Temperierung des Gemisches auf eine zur Bildung einer stabilen Iminverbindung der Formel RCH=NR' ausreichend hohen Temperatur in Gegenwart einer Wasserstoffquelle und eines rhodiumbeladenen Hydrierungskatalysators.

2. Verfahren nach Anspruch 1, wobei der rhodiumbeladene Katalysator Rhodium auf Kohlenstoff ist.

3. Verfahren nach Anspruch 2, wobei der rhodiumbeladene Katalysator etwa 5 % Rhodium auf Kohlenstoff ist.

4. Verfahren nach Anspruch 1, wobei das primäre Amin ein aromatisches Amin ist.

5. Verfahren nach Anspruch 1, wobei das primäre Amin ein aliphatisches Amin ist.

6. Verfahren nach Anspruch 1, welches außerdem die Schritte der Abtrennung und der Wiedergewinnung des Hydrierungskatalysators umfaßt.

7. Verfahren zur Herstellung einer unsymmetrischen sekundären Aminverbindung der Formel RCH$_2$NHR$^1$, wobei R eine 3-Pyridylgruppe und R$^1$ eine aliphatische oder aromatische Gruppe ist mit den Schritten:

Zur Reaktion bringen eines Gemisches von 3-Cyanopyridin und einem ersten primären Amin mittels des Verfahrens nach Anspruch 1 zur Erzeugung eines ersten stabilen Iminzwischenproduktes;

Zusammenbringen des stabilen Iminzwischenproduktes mit einem zweiten primären Amin der Formel R$^2$NH$_2$, wobei R$^2$ eine aromatische Gruppe ist, und veranlassen, daß das so gebildete Gemisch auf einer hinreichend hohen Temperatur und/oder hinreichend sauer ist, damit sich ein zweites stabiles Iminzwischenprodukt bildet; und

Zusammenbringen des zweiten stabilen Iminzwischenproduktes mit einer Wasserstoffquelle in Gegenwart eines geeigneten Hydrierungskatalysators bei erhöhtem Druck und bei einer Temperatur, die hinreichend hoch ist, daß sich dabei die sekundäre Aminverbindung der Formel RCH$_2$NHR$^2$ bildet.

8. Verfahren nach Anspruch 7, wobei das zweite primäre Amin ein Polyhalogenanilin ist.

9. Verfahren nach Anspruch 8, wobei das Polyhalogenanilin ein Polyfluoroanilin ist.

10. Verfahren nach Anspruch 9, wobei das Polyfluoroanilin 2,4-Difluoroanilin ist.

11. Verfahren nach Anspruch 7, wobei der rhodiumbeladene Hydrierungskatalysator Rhodium auf Kohlenstoff ist.

12. Verfahren nach Anspruch 7, wobei die Hydrierung des zweiten Iminzwischenproduktes in Gegenwart eines Hydrierungskatalysators aus der Gruppe, bestehend aus Platin auf Kohlenstoff, Palladium auf Kohlenstoff oder Bariumsulfat, Rhodium auf Kohlenstoff und Rhodium auf Aluminiumoxid ist.

13. Verfahren nach Anspruch 7 mit den weiteren Schritten der Abtrennung und Wiedergewinnung des rhodiumbeladenen Hydrierungskatalysators nach der Hydrierung, jedoch vor der Transiminierung.

14. Verfahren nach Anspruch 13, wobei die Hydrierung in Gegenwart eines Lösungsmittels geschieht.

15. Verfahren nach Anspruch 14, wobei das Lösungsmittel das erste primäre Amin ist.

16. Verfahren nach Anspruch 14, mit dem weiteren Schritt der Entfernung des Lösungsmittels nach der Abtrennung und Wiedergewinnung und vor der Transiminierung.

17. Verfahren nach Anspruch 16, wobei die Transiminierung in Gegenwart von Salzsäure geschieht.

18. Verfahren nach Anspruch 7, wobei das erste primäre Amin ein primäres aliphatisches Amin ist.

19. Verfahren zur Herstellung von 3-Pyridylaldehyd mit den Schritten:

Bildung eines Gemisches eines 3-Cyanopyridins und eines primären Amins und zur Reaktion bringen des Gemisches durch das Verfahren nach Anspruch 1 zur Bildung eines stabilen Iminzwischenproduktes der Formel RCH=NR', wobei R eine 3-Pyridylgruppe ist;

Abtrennung des Hydrierungskatalysators von dem Iminzwischenprodukt; und

Hydrolyse des Iminzwischenproduktes zu einem entsprechenden 3-Pyridylaldehyd.

20. Verfahren nach Anspruch 19, wobei der rhodiumbeladene Katalysator Rhodium auf Kohlenstoff ist.

21. Verfahren nach Anspruch 20, wobei der rhodiumbeladene Katalysator etwa 5 % Rhodium auf Kohlenstoff ist.

22. Verfahren nach Anspruch 19, wobei das primäre Amin ein aliphatisches Amin ist.

23. Verfahren nach Anspruch 22, wobei die Hydrierung in Gegenwart eines Lösungsmittels geschieht.

24. Verfahren nach Anspruch 23, wobei das Lösungsmittel das primäre Amin ist.

**Revendications**

1. Procédé pour préparer une composition à base d'imines ayant la formule RCH=NR' dans laquelle R est un groupe 3-pyridyle et R' est un groupe aliphatique ou aromatique, comprenant l'étape de :

   • former un mélange composé d'une 3-cyanopyridine et d'une amine primaire de formule $R'NH_2$ ;
   • permettre au mélange d'être à une température suffisamment élevée en présence d'une source d'hydrogène et d'un catalyseur d'hydrogénation chargé en rhodium pour former une composition à base d'imines stables de formule RCH=NR'.

2. Procédé selon la revendication 1,
   dans lequel
   le catalyseur chargé en rhodium est du rhodium sur carbone.

3. Procédé selon la revendication 2,
   dans lequel
   le catalyseur chargé en rhodium est du rhodium à peu près à 5 % sur carbone.

4. Procédé selon la revendication 1,
   dans lequel
   l'amine primaire est une amine aromatique.

5. Procédé selon la revendication 1,
   dans lequel
   l'amine primaire est une amine aliphatique.

6. Procédé selon la revendication 1 et plus comprenant les étapes de séparation et de récupération du catalyseur d'hydrogénation.

7. Procédé de préparation d'une composition à base d'amines secondaires non symétriques de formule $RCH_2NHR^1$ où R est un groupe 3-pyridyle et $R^1$ est un groupe aliphatique ou aromatique, comprenant les étapes de :

   • permettre à un mélange composé de 3-pyridine et d'une première amine primaire de réagir par le procédé selon la revendication 1 pour produire un premier intermédiaire imine stable ;
   • mettre en contact l'intermédiaire imine stable avec une seconde amine primaire de formule $R^2NH_2$, où $R^2$ est un groupe aromatique, et permettre au mélange ainsi formé d'être à une température suffisamment élevée et/ou d'être suffisamment acide pour produire un second intermédiaire imine stable ; et
   • mettre en contact le second intermédiaire imine stable avec une source d'hydrogène en présence d'un catalyseur d'hydrogénation convenable sous pression élevée et à une température suffisamment élevée pour former de ce fait la composition à base d'amines secondaires de formule $RCH_2NHR^2$.

8. Procédé selon la revendication 7,
   dans lequel
   la seconde amine primaire est une polyhaloaniline.

9. Procédé selon la revendication 8
   dans lequel
   la polyhaloaniline est une polyfluoroaniline.

10. Procédé selon la revendication 9,
    dans lequel
    la polyfluoroaniline est une 2,4-difluoroaniline.

11. Procédé selon la revendication 7,
    dans lequel
    le catalyseur d'hydrogénation chargé en rhodium est du rhodium sur carbone.

12. Procédé selon la revendication 7,
    dans lequel
    cette hydrogénation du second intermédiaire imine s'effectue en présence d'un catalyseur d'hydrogénation choisi parmi le groupe constitué de platine sur carbone, de palladium sur carbone ou de sulfate de baryum, de rhodium sur carbone, et de rhodium sur oxyde d'aluminium.

13. Procédé selon la revendication 7 et plus comprenant les étapes de séparation et de récupération du catalyseur d'hydrogénation chargé en rhodium après la hydrogénation mais avant la transimination.

14. Procédé selon la revendication 13,
    dans lequel
    la hydrogénation s'effectue en présence d'un solvant.

15. Procédé selon la revendication 14,
    dans lequel
    le solvant est la première amine primaire.

16. Procédé selon la revendication 14 et plus comprenant l'étape d'élimination du solvant après la séparation et récupération et avant la transimination.

17. Procédé selon la revendication 16,
    dans lequel
    la transimination s'effectue en présence d'un acide hydrochlorique.

18. Procédé selon la revendication 7,
    dans lequel
    la première amine primaire est une amine aliphatique.

19. Procédé pour préparer une 3-pyridylaldéhyde comprenant les étapes de :

    • former un mélange d'une 3-cyanopyridine et d'une amine primaire et leur permettre de réagir par le procédé selon la revendication 1, pour former un intermédiaire imine stable de formule RCH=NR' dans lequel R est un groupe 3-pyridyle ;
    • séparer le catalyseur d'hydrogénation de l'intermédiaire imine ; et
    • hydrolyser l'intermédiaire imine en 3-pyridylaldéhyde correspondant.

20. Procédé selon la revendication 19,
    dans lequel
    le catalyseur chargé en rhodium est du rhodium sur carbone.

21. Procédé selon la revendication 20,
    dans lequel
    le catalyseur chargé en rhodium est du rhodium à peu près à 5 % sur carbone.

22. Procédé selon la revendication 19,
    dans lequel
    l'amine primaire est une amine aliphatique.

23. Procédé selon la revendication 22,
    dans lequel
    la hydrogénation s'effectue en présence d'un solvant.

24. Procédé selon la revendication 23,
    dans lequel
    le solvant est une amine primaire.